(19) Europäisches Patentamt
European Patent Office
Office européen des brevets

(11) Veröffentlichungsnummer: **0 302 334 A2**

(12) **EUROPÄISCHE PATENTANMELDUNG**

(21) Anmeldenummer: **88111927.5**

(22) Anmeldetag: **25.07.88**

(51) Int. Cl.4: **C07F 9/141 , C07F 9/15 , C07F 9/165 , C07F 9/21 , C07F 9/40 , A01N 57/02 , A01N 57/36**

(30) Priorität: **04.08.87 DE 3725849**

(43) Veröffentlichungstag der Anmeldung: **08.02.89 Patentblatt 89/06**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Andree, Roland, Dr.**
**Schillerstrasse 17**
**D-4018 Langenfeld(DE)**
Erfinder: **Förster, Heinz, Dr.**
**Am Eckbusch 47**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6A**
**D-4000 Düsseldorf 31(DE)**

(54) **(Thio)Phosphoryloxyessigsäureamide.**

(57) Die Erfindung betrifft neue (Thio)Phosphoryloxyessigsäureamide der allgemeinen Formel (I)

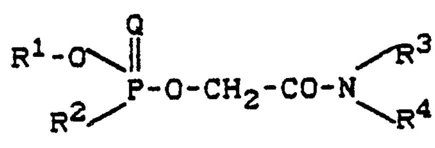

(I)

in welcher
Q für Sauerstoff oder Schwefel steht,
$R^1$ für gegebenenfalls durch Halogen substituiertes $C_1$-$C_6$-Alkyl oder Phenyl steht,
$R^2$ für gegebenenfalls durch Halogen substituiertes $C_1$-$C_6$-Alkyl oder Phenyl, für gegebenenfalls durch Halogen substituiertes $C_1$-$C_6$-Alkoxy oder Phenoxy oder für gegebenenfalls durch Halogen substituiertes $C_1$-$C_6$-Alkylthio oder Phenylthio steht, oder die beiden Gruppierungen
O-$R^1$ und $R^2$ zusammen für gegebenenfalls verzweigtes $C_2$-$C_5$-Alkylendioxy stehen,
$R^3$ für gegebenenfalls durch Halogen oder $C_1$-$C_4$-Alkoxy substituiertes $C_1$-$C_6$-Alkyl, für gegebenenfalls durch Halogen substituiertes $C_2$-$C_6$-Alkenyl oder für $C_2$-$C_6$-Alkinyl steht und
$R^4$ für gegebenenfalls durch Halogen oder $C_1$-$C_4$-Alkoxy substituiertes $C_1$-$C_6$-Alkyl, für gegebenenfalls

EP 0 302 334 A2

durch Halogen substituiertes $C_2$-$C_6$-Alkenyl, für $C_2$-$C_6$-Alkinyl, für $C_5$- oder $C_6$-Cycloalkenyl, für gegebenenfalls durch $C_1$-$C_4$-Alkoxy substituiertes $C_1$-$C_6$-Alkoxy, für $C_3$-$C_4$-Alkenyloxy oder für durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio und/oder $C_1$-$C_2$-Halogenalkylthio substituiertes Phenyl steht,
ein Verfahren zu ihrer Herstellung und ihre Verwendung als Herbizide.

## (Thio)Phosphoryloxyessigsäureamide

Die Erfindung betrifft neue (Thio)Phosphoryloxyessigsäureamide, ein Verfahren zu ihrer Herstellung und ihre Verwendung als Herbizide.

Es ist bekannt, daß bestimmte Dialkoxythiophosphorylmercaptoessigsäureanilide, wie z. B. N-(4-Chlorphenyl)-N-isopropyl-S-dimethoxythiophosphoryl-mercaptoessigsäureamid (Anilofos) herbizide Eigenschaften aufweisen (vgl. DE-OS 26 04 224). Die herbizide Wirksamkeit dieser vorbekannten Verbindungen gegenüber Schadpflanzen bzw. ihre Selektivität in Nutzpflanzen ist jedoch nicht immer ganz zufriedenstellend.

Es wurden nun neue (Thio)Phosphoryloxyessigsäureamide der allgemeinen Formel (I)

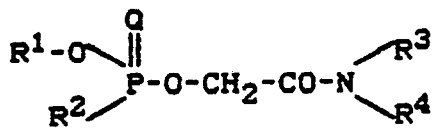

(I)

in welcher
Q für Sauerstoff oder Schwefel steht,
$R^1$ für gegebenenfalls durch Halogen substituiertes $C_1$-$C_6$-Alkyl oder Phenyl steht,
$R^2$ für gegebenenfalls durch Halogen substituiertes $C_1$-$C_6$-Alkyl oder Phenyl, für gegebenenfalls durch Halogen substituiertes $C_1$-$C_6$-Alkoxy oder Phenoxy oder für gegebenenfalls durch Halogen substituiertes $C_1$-$C_6$-Alkylthio oder Phenylthio steht, oder die beiden Gruppierungen
O-$R^1$ und $R^2$ zusammen für gegebenenfalls verzweigtes $C_2$-$C_5$-Alkylendioxy stehen,
$R^3$ für gegebenenfalls durch Halogen oder $C_1$-$C_4$-Alkoxy substituiertes $C_1$-$C_6$-Alkyl, für gegebenenfalls durch Halogen substituiertes $C_2$-$C_6$-Alkenyl oder für $C_2$-$C_6$-Alkinyl steht und
$R^4$ für gegebenenfalls durch Halogen oder $C_1$-$C_4$-Alkoxy substituiertes $C_1$-$C_6$-Alkyl, für gegebenenfalls durch Halogen substituiertes $C_2$-$C_6$-Alkenyl, für $C_2$-$C_6$-Alkinyl, für $C_5$- oder $C_6$-Cycloalkenyl, für gegebenenfalls durch $C_1$-$C_4$-Alkoxy substituiertes $C_1$-$C_6$-Alkoxy, für $C_3$-$C_4$-Alkenyloxy oder für durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio und/oder $C_1$-$C_2$-Halogenalkylthio substituiertes Phenyl steht,
gefunden.

Weiter wurde gefunden, daß man die neuen (Thio)Phosphoryloxyessigsäureamide der allgemeinen Formel (I) erhält, wenn man (Thio)Phosphorylhalogenide der allgemeinen Formel (II)

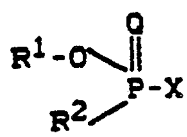

(II)

in welcher
Q, $R^1$ and $R^2$ die oben angegebenen Bedeutungen haben
und
X für Halogen steht,
mit Hydroxyessigsäureamiden der allgemeinen Formel (III)

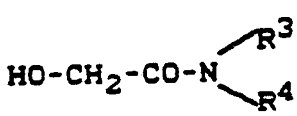

(III)

in welcher
$R^3$ und $R^4$ die oben angegebenen Bedeutungen haben,
gegebenenfalls in Gegenwart eines Verdünnungsmittels und gegebenenfalls in Gegenwart eines Säurebindemittels umsetzt.

Schließlich wurde gefunden, daß die neuen (Thio)Phosphoryloxyessigsäureamide der allgemeinen

Formel (I) interessante herbizide Eigenschaften besitzen.

Überraschenderweise zeigen die erfindungsgemäßen (Thio)Phosphoryloxyessigsäureamide der Formel (I) erheblich stärkere herbizide Wirkung gegen verbreitete, schwer bekämpfbare Unkräuter als das bekannte N-(4-Chlor-phenyl)-N-isopropyl-S-dimethoxythiophosphoryl-mercaptoessigsäureamid, welches eine nach Struktur und Wirkprofil vergleichbare Verbindung ist.

Gegenstand der Erfindung sind vorzugsweise Verbindungen der Formel (I), in welcher

Q für Sauerstoff oder Schwefel steht,

$R^1$ für gegebenenfalls durch Fluor und/oder Chlor substituiertes $C_1$-$C_4$-Alkyl oder Phenyl steht,

$R^2$ für gegebenenfalls durch Fluor und/oder Chlor substituiertes $C_1$-$C_4$-Alkyl oder Phenyl, für gegebenenfalls durch Fluor und/oder Chlor substituiertes $C_1$-$C_4$-Alkoxy oder Phenoxy oder für gegebenenfalls durch Fluor und/oder Chlor substituiertes $C_1$-$C_4$-Alkylthio oder Phenylthio steht, oder die beiden Gruppierungen

O-$R^1$ und $R^2$ zusammen für Ethan-1,2-dioxy (Ethylendioxy) oder Propan-1,3-dioxy (Propylendioxy) stehen,

$R^3$ für gegebenenfalls durch Fluor, Chlor oder $C_1$-$C_3$-Alkoxy substituiertes $C_1$-$C_4$-Alkyl, für gegebenenfalls durch Fluor oder Chlor substituiertes $C_3$-$C_4$-Alkenyl oder für $C_3$-$C_4$-Alkinyl steht und

$R^4$ für gegebenenfalls durch Fluor, Chlor oder $C_1$-$C_3$-Alkoxy substituiertes $C_1$-$C_4$-Alkyl, für gegebenenfalls durch Fluor oder Chlor substituiertes $C_3$-$C_4$-Alkenyl, für $C_3$-$C_4$-Alkinyl, für $C_5$- oder $C_6$-Cycloalkenyl, für gegebenenfalls durch $C_1$-$C_3$-Alkoxy substituiertes $C_1$-$C_4$-Alkoxy, für $C_3$-$C_4$-Alkenyloxy oder für durch Fluor, Chlor, Brom, $C_1$-$C_3$-Alkyl, Trifluormethyl, $C_1$-$C_3$-Alkoxy, $C_1$-$C_2$-Fluoralkoxy, $C_1$-$C_2$-Chlorfluoralkoxy, $C_1$-$C_3$-Alkylthio, $C_1$-$C_2$-Fluoralkylthio und/oder $C_1$-$C_2$-Chlorfluoralkylthio substituiertes Phenyl steht.

Die Erfindung betrifft insbesondere Verbindungen der Formel (I), in welcher

Q für Sauerstoff oder Schwefel steht,

$R^1$ für Methyl, Ethyl, Propyl oder Isopropyl steht,

$R^2$ für Methoxy, Ethoxy, Propoxy, Isopropoxy, Methylthio, Ethylthio, Propylthio oder Isopropylthio steht, oder die beiden Gruppierungen

O-$R^1$ und $R^2$ zusammen für Propan-1,3-dioxy (Propylendioxy) stehen,

$R^3$ für Methyl, Ethyl, Propyl, Isopropyl, Butyl, Isobutyl, Methoxymethyl, Methoxyethyl, Methoxypropyl, Ethoxymethyl, Propoxymethyl, Isopropoxymethyl, Ethoxyethyl, Propoxyethyl, Isopropoxyethyl, Allyl oder Propargyl steht und

$R^4$ für Methyl, Ethyl, Propyl, Isopropyl, Butyl, Isobutyl, Methoxyethyl, Ethoxyethyl, Propoxyethyl, Isopropoxyethyl, Allyl, Propargyl, Cyclohexenyl, Methoxy, Ethoxy, Propoxy, Isopropoxy, Butoxy, Isobutoxy, Methoxyethoxy, Ethoxyethoxy oder für durch Fluor, Chlor, Brom, Methyl, Ethyl, Trifluormethyl, Methoxy, Ethoxy, Difluormethoxy, Trifluormethoxy, Chlordifluormethoxy, Methylthio, Ethylthio, Trifluormethylthio und/oder Chlordifluormethylthio substituiertes Phenyl steht.

Beispiele für ganz besonders bevorzugte Verbindungen der Formel (I) sind in der nachstehenden Tabelle 1 aufgeführt.

**Tabelle 1: Beispiele für Verbindungen der Formel (I)**

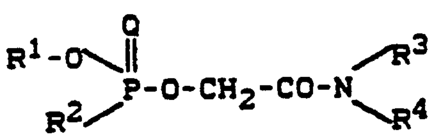

(I)

Tabelle 1

| Q | $R^1$ | $R^2$ | $R^3$ | $R^4$ |
|---|---|---|---|---|
| S | $CH_3$ | $OCH_3$ | $CH(CH_3)_2$ | 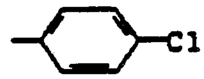 |
| S | $C_2H_5$ | $OC_2H_5$ | $CH(CH_3)_2$ | 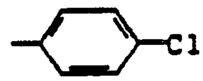 |
| S | $CH_3$ | $OCH_3$ | $CH_3$ | 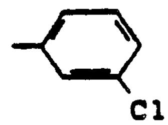 |
| S | $C_2H_5$ | $OC_2H_5$ | $CH_3$ | 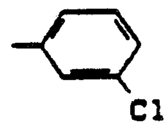 |
| S | $CH_3$ | $OC_3H_7$ | $CH(CH_3)_2$ | 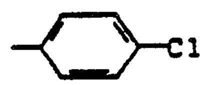 |
| S | $CH_3$ | $OCH_3$ | $C_2H_5$ | 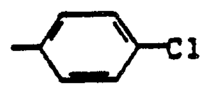 |
| S | $C_2H_5$ | $OC_2H_5$ | $C_2H_5$ | 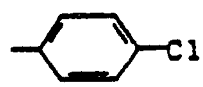 |
| S | $CH_3$ | $OC_2H_5$ | $C_3H_7$ | 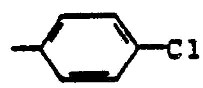 |
| S | $C_2H_5$ | $OC_3H_7$ | $CH_3$ | 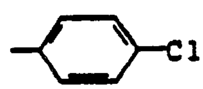 |
| S | $C_2H_5$ | $OCH(CH_3)_2$ | $CH_3$ | 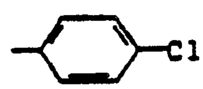 |

Tabelle 1 - Fortsetzung

| Q | $R^1$ | $R^2$ | $R^3$ | $R^4$ |
|---|---|---|---|---|
| S | $CH_3$ | $OCH_3$ | $CH(CH_3)_2$ | 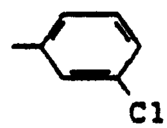 |
| S | $C_2H_5$ | $OC_2H_5$ | $CH(CH_3)_2$ | 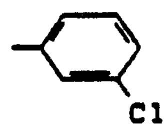 |
| S | $C_2H_5$ | $OCH(CH_3)_2$ | $CH(CH_3)_2$ | 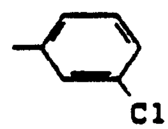 |
| S | $CH_3$ | $OCH_3$ | $CH(CH_3)_2$ | 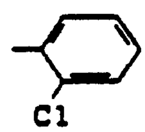 |
| S | $C_2H_5$ | $OC_2H_5$ | $CH(CH_3)_2$ | 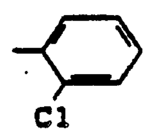 |
| S | $CH_3$ | $OCH_3$ | $C_2H_5$ | 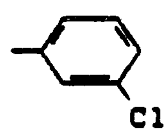 |
| S | $C_2H_5$ | $OC_2H_5$ | $C_2H_5$ | 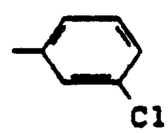 |

Verwendet man beispielsweise Phosphorsäurechloriddimethylester und Hydroxyessigsäure-diethylamid als Ausgangsstoffe, so läßt sich der Reaktionsablauf beim erfindungsgemäßen Verfahren durch das folgende Formelschema darstellen:

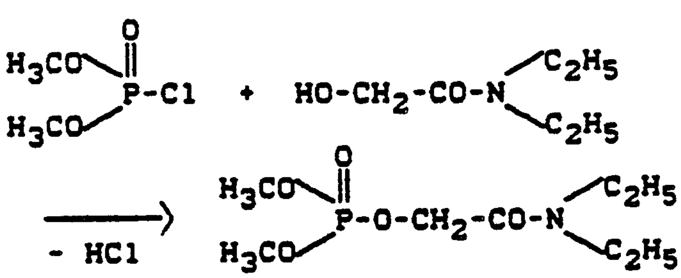


Die zur Durchführung des erfindungsgemäßen Verfahrens als Ausgangsstoffe zu verwendenden (Thio)-Phosphorylhalogenide sind durch die Formel (II) allgemein definiert. In Formel (II) haben Q, $R^1$ und $R^2$ vorzugsweise bzw. insbesondere diejenigen Bedeutungen, die bereits oben im Rahmen der Beschreibung der erfindungsgemäßen Verbindungen der Formel (I) als bevorzugt bzw. als insbesondere bevorzugt für Q,

$R^1$ und $R^2$ angegeben wurden und X steht vorzugsweise für Fluor, Chlor oder Brom, insbesondere für Chlor.

Als Beispiele für die Ausgangsstoffe der Formel (II) seien genannt:

Methan-, Ethan-, und Benzol-phosphonsäure-chlorid-methylester, -ethylester, -propylester, -butylester, -pentylester und -hexylester, Methan-, Ethan- und Benzol-thiophosphonsäure-chlorid-S-methylester, -S-ethylester, -S-propylester, -S-butylester, -S-pentylester und -S- hexylester, Methan-, Ethan- und Benzol-thiophosphonsäure-chlorid-O-methylester, -O-ethylester, -O-propylester, -O-butylester, -O-pentylester und -O-hexylester, Phosphorsäure-chlorid-dimethylester, -diethylester, -dipropylester, -dibutylester, -methylester-ethylester, -methylester-propylester, -methylester-butylester, -ethylester-propylester, -ethylester-butylester und -propylester-butylester, Thiophosphorsäure-chlorid-O,S-dimethylester, -O,S-diethylester, -O,S-dipropylester, -O,S-dibutylester, -O-methylester-S-ethylester, -O-methylester-S-propylester, -O-methylester-S-butylester, -O-ethylester-S-methylester, -O-ethylester-S-propylester, -O-ethylester-S-butylester, -O-propylester-S-methylester, -O-propylester-S-ethylester, -O-propylester-S-butylester, -O-butylester-S-methylester, -O-butylester-S-ethylester und -O-butylester-S-propylester, Thiophosphorsäure-chlorid-O,O-dimethylester, -O,O-diethylester, -O,O-dipropylester, -O,O-dibutylester, -O-methylester-O-ethylester, -O-methylester-O-propylester, -O-methylester-O-butylester, -O-ethylester-O-propylester, -O-ethylester-O-butylester und -O-propylester-O-butylester, Dithiophosphorsäure-chlorid-O,S-dimethylester, -O,S-diethylester, -O,S-dipropylester, -O,S-dibutylester, -O-methylester-S-ethylester, -O-methylester-S-propylester, -O-methylester-S-butylester, -O-ethylester-S-propylester, -O-ethylester-S-butylester, -O-ethylester-S-methylester, -O-propylester-S-methylester, -O-propylester-S-ethylester, -O-propylester-S-butylester, -O-butylester-S-methylester, -O-butylester-S-ethylester und -O-butylester-S-propylester.

Die Ausgangsstoffe der Formel (II) sind bekannt (vgl. Houben-Weyl, Methoden der organischen Chemie, Band 12/1, S. 415 - 420 und S. 560 - 563, Band 12/2, S. 274 - 292, S. 405 - 408, S. 607 - 618, S. 621 - 622 und S. 755 -757, Band E2, S. 333 - 684).

Die weiter als Ausgangsstoffe zu verwendenden Hydroxyessigsäureamide sind durch die Formel (III) allgemein definiert. In Formel (III) haben $R^3$ und $R^4$ vorzugsweise bzw. insbesondere diejenigen Bedeutungen, die bereits oben im Rahmen der Beschreibung der erfindungsgemäßen Verbindungen der Formel (I) als bevorzugt bzw. als insbesondere bevorzugt für $R^3$ bzw. $R^4$ angegeben wurden.

Beispiele für die Ausgangsstoffe der Formel (III) sind in der nachstehenden Tabelle 2 aufgeführt.

**Tabelle 2: Beispiele für Verbindungen der Formel (III)**

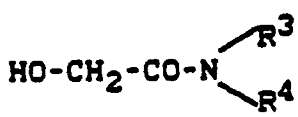

(III)

| $R^3$ | $R^4$ | $R^3$ | $R^4$ |
|---|---|---|---|
| $CH_3$ | $CH_3$ | $C_2H_5$ | $C_2H_5$ |
| $C_3H_7$ | $C_3H_7$ | $CH(CH_3)_2$ | $CH(CH_3)_2$ |
| $CH_3$ | $C_3H_7$ | $CH_3$ | $CH(CH_3)_2$ |
| $CH_3$ | $C_4H_9$ | $CH_3$ | $CH_2CH(CH_3)_2$ |
| $CH_3$ | $\underset{CH_3}{\underset{\vert}{CH}}CH_2CH_3$ | $CH_2CH=CH_2$ | $CH_2CH=CH_2$ |

Tabelle 2 - Fortsetzung

| $R^3$ | $R^4$ | $R^3$ | $R^4$ |
|---|---|---|---|
| $CH_2C\equiv CH$ | $CH_2C\equiv CH$ | $C_4H_9$ | $C_4H_9$ |
| $CH_2CH(CH_3)_2$ | $CH_2CH(CH_3)_2$ | $CH_2CH_2OCH_3$ | $CH_2CH_2OCH_3$ |
| $CH_2CH_2OC_2H_5$ | $CH_2CH_2OC_2H_5$ | $CH_3$ | $OCH_3$ |
| $CH_3$ | $OC_2H_5$ | $C_2H_5$ | $OCH_3$ |
| $C_2H_5$ | $OC_2H_5$ | $CH_3$ | $OC_3H_7$ |
| $C_3H_7$ | $OCH_3$ | $C_3H_7$ | $OC_3H_7$ |
| $CH_3$ | $OCH(CH_3)_2$ | $CH(CH_3)_2$ | $OCH_3$ |
| $CH_3$ | $OCH_2CH_2OCH_3$ | $CH(CH_3)_2$ | $OCH_2CH_2OCH_3$ |
| $CH_3$ | $OCH_2CH_2OC_2H_5$ | $CH(CH_3)_2$ | $OCH_2CH_2OC_2H_5$ |
| $C_2H_5$ | $OCH_2CH_2OC_2H_5$ | $CH_3$ | $OC_4H_9$ |
| $CH_3$ | $OCH_2CH(CH_3)_2$ | $CH_3$ | 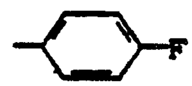 |
| $CH_3$ | 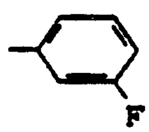 | $CH_3$ | 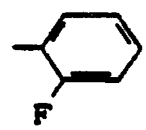 |
| $CH_3$ | 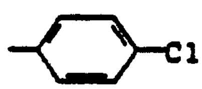 | $CH_3$ | 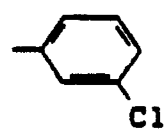 |
| $CH_3$ | 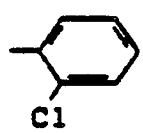 | $CH_3$ | 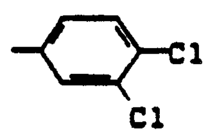 |

Tabelle 2 - Fortsetzung

| $R^3$ | $R^4$ | $R^3$ | $R^4$ |
|---|---|---|---|
| $CH_3$ | 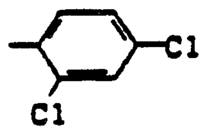 | $CH_3$ | 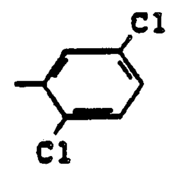 |
| $CH_3$ | 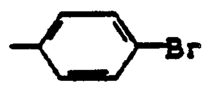 | $CH_3$ | 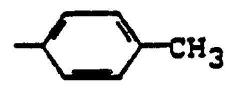 |
| $CH_3$ | 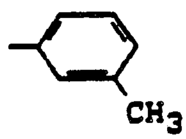 | $CH_3$ | 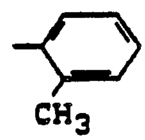 |
| $CH_3$ | 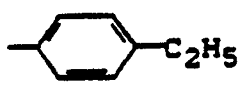 | $CH_3$ | 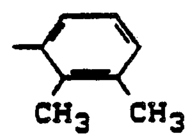 |
| $CH_3$ | 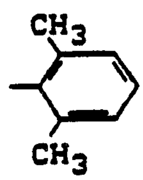 | $CH_3$ | 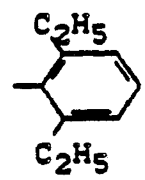 |
| $CH_2OCH_3$ | 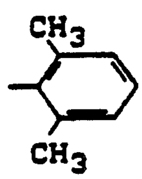 | $CH_2OCH_3$ | 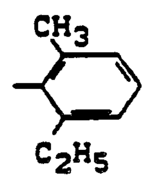 |
| $CH_2OCH_3$ | 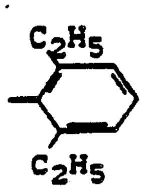 | $CH(CH_3)CH_2OCH_3$ | 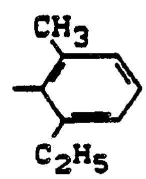 |
| $CH_2CH_2OCH_3$ | 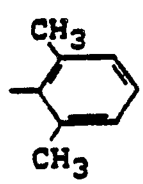 | $CH_3$ | 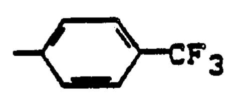 |

Tabelle 2 - Fortsetzung

| $R^3$ | $R^4$ | $R^3$ | $R^4$ |
|---|---|---|---|
| $CH_3$ | 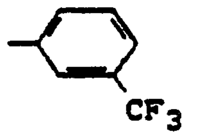 | $CH_3$ | 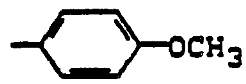 |
| $CH_3$ | 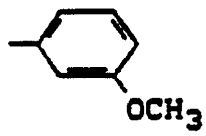 | $CH_3$ | 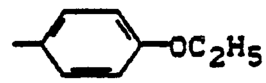 |
| $CH_3$ | 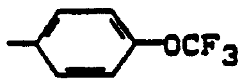 | $CH_3$ | 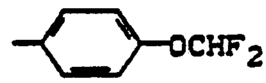 |
| $CH_3$ | 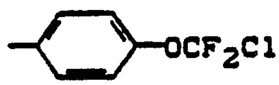 | $CH_3$ | 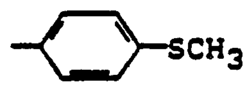 |
| $CH_3$ | 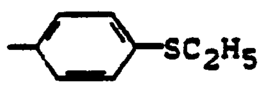 | $CH_3$ | 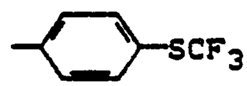 |
| $CH_3$ | 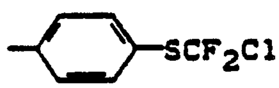 | $C_2H_5$ | 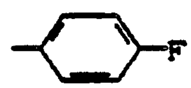 |
| $C_2H_5$ | 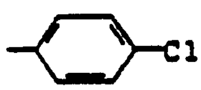 | $C_2H_5$ | 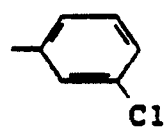 |
| $C_2H_5$ | 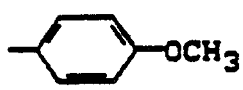 | $C_2H_5$ | 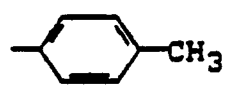 |
| $C_3H_7$ | 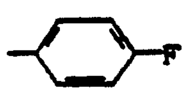 | $C_3H_7$ | 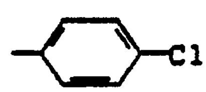 |
| $C_3H_7$ | 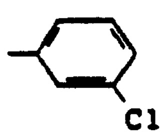 | $C_3H_7$ | 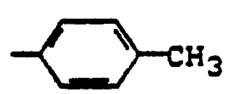 |

Tabelle 2 - Fortsetzung

| $R^3$ | $R^4$ | $R^3$ | $R^4$ |
|---|---|---|---|
| $C_3H_7$ | 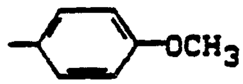 | $CH(CH_3)_2$ | 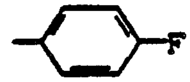 |
| $CH(CH_3)_2$ | 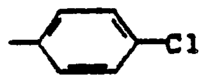 | $CH(CH_3)_2$ | 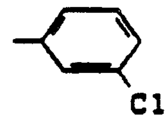 |
| $CH(CH_3)_2$ | 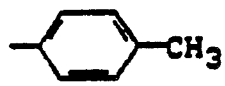 | $CH(CH_3)_2$ | 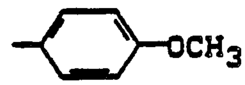 |
| $CH_3$ | 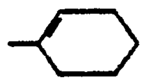 | $CH_3$ | $CH_2OCH_3$ |
| $CH_3$ | 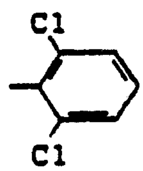 | $CH(CH_3)_2$ | 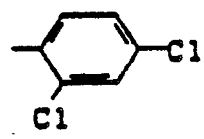 |
| $CH(CH_3)_2$ | 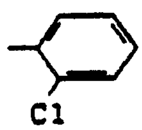 | $CHCH_2CH_3$ (mit $CH_3$) | $OCH_3$ |
| $CH(CH_3)_2$ | 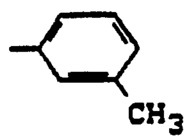 | $CH(CH_3)_2$ | 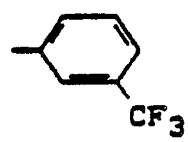 |
| $C_4H_9$ | 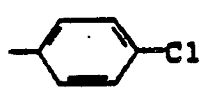 | $CH_2CH(CH_3)_2$ | 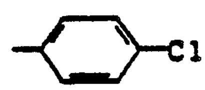 |

Die Hydroxyessigsäureamide der Formel (III) sind bekannt und/oder können nach an sich bekannten Verfahren hergestellt werden (vgl. US-A 4 509 971 und US-A 4 645 525; ferner US-A-4 334 073, DE-A-3 038 598, DE-A-3 038 636).

Das erfindungsgemäße Verfahren zur Herstellung der neuen (Thio)Phosphoryloxyessigsäureamide der Formel (I) wird vorzugsweise unter Verwendung von Verdünnungsmitteln durchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether, Diisopropylether, Dibutylether, Diisobutylether, Glycoldimethylether, Tetrahydrofuran und Dioxan, Alkohole, wie z. B. Methanol, Ethanol, Propanol, Isopropanol oder Butanol, Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamid, Dimethylacetamid und N-Methyl-pyrrolidon, Nitrile, wie z. B. Acetonitril und Propionitril, Sulfoxide, wie z. B. Dimethylsulfoxid sowie Wasser.

Das erfindungsgemäße Verfahren wird vorzugsweise unter Verwendung von Säurebindemitteln durchgeführt. Als solche kommen vorzugsweise in Betracht Alkalimetall- bzw. Erdalkalimetall-hydroxide, wie z. B. Lithium-, Natrium-, Kalium und Calcium-hydroxid, Erdalkalimetalloxide, wie z. B. Magnesium- und Calciumoxid, Alkalimetall- bzw. Erdalkalimetall-carbonate, wie z. B. Natrium-, Kalium- und Calcium-carbonat, sowie Alkalimetallalkoholate, wie z. B. Natrium- und Kalium-methylat, -ethylat und -tert-butylat.

Die Reaktionstemperaturen können bei dem erfindungsgemäßen Verfahren in einem größeren Bereich variiert werden. Im allgemeinen arbeitet man bei Temperaturen zwischen -50 °C und +100 °C, vorzugsweise bei Temperaturen zwischen -20 °C und +100 °C.

Das erfindungsgemäße Verfahren wird im allgemeinen bei Normaldruck durchgeführt; es kann aber auch bei erhöhtem oder vermindertem Druck, etwa zwischen 0,1 und 10 bar, durchgeführt werden.

Zur Durchführung des erfindungsgemäßen Verfahrens setzt man je Mol (Thio)Phosphorylhalogenid der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammen gegeben werden. Vorzugsweise werden das Hydroxyessigsäureamid, das Säurebindemittel und das Verdünnungsmittel zuerst vermischt und das (Thio)Phosphorylhalogenid wird dann langsam dazugegeben. Das Reaktionsgemisch wird bis zum Ende der Umsetzung gerührt und dann nach üblichen Methoden aufgearbeitet. Vorzugsweise wird - gegebenenfalls nach Einengen - mit einem mit Wasser praktisch nicht mischbaren Lösungsmittel, wie z. B. Methylenchlorid, verdünnt, mit Wasser gewaschen, getrocknet und filtriert. Der nach Abdestillieren des Lösungsmittels vom Filtrat verbleibende Rückstand enthält im wesentlichen das Produkt der Formel (I). Eine Reinigung ist nach üblichen Methoden, beispielsweise durch Säulenchromatographie und/oder Umkristallisation möglich.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u>

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u>

Echinochloa, Seta ria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung monokotyler Unkräuter in dikotylen Kulturen im Vorauflaufverfahren. Sie sind bei gleich guter Verträglichkeit für Zuckerrüben, Soja und Baumwolle deutlich wirksamer als der bekannte Wirkstoff Anilofos. Die erfindungsgemäßen Verbindungen zeigen auch Wirkung gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser. Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Ge steinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylakohol, Polyvinylacetat, sowie natürliche Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage, ferner auch 5-Amino-4-chlor-2-phenyl-2,3-dihydro-3-oxy-pyridazin; 1-(3-Trifluormethyl-phenyl)-4-methylamino-5-chlor-6-pyridazon; Chloressigsäure-N-(methoxymethyl)-2,6-diethylanilid; N-(Butoxymethyl)-2-chlor-N-(2,6-diethylphenyl)-acetamid; 2-Chlor-N-(2,6-dimethylphenyl)-N-[(1H)-pyrazol-1-yl-methyl]-acetamid;

2-Ethyl-6-methyl-N-(1-methyl-2-methoxyethyl)-chloracetanilid; α-Chlor-2′,6′-diethyl-N-(2-propoxyethyl)-acetanilid; 2-Chlor-N-isopropylacetanilid; N-(1-Ethylpropyl)-3,4-dimethyl-2,6-dinitroanilin; 2,6-Dinitro-4-trifluormethyl-N,N-dipropylanilin; Methyl-2-[4,5-dihydro-4-methyl-4-(1-methylethyl)-5-oxo-1H-imidazol-2-yl]-4-(5)-methylbenzoat; 2-[5-Methyl-5-(1-methylethyl)-4-oxo-2-imidazolin-2-yl]-3-chinolincarbonsäure; N,N-Dimethyl-N′-(3-trifluormethylphenyl)-harnstoff; 2-[4,5-Dihydro-4-methyl-4-isopropyl-5-oxo-(1H)-imidazol-2-yl]-5-ethyl-pyridin-3-carbonsäure; N-Methyl-2-(1,3-benzthiazol-2-yloxy)-acetanilid; 2-[[[[(4,6-Dimethoxypyri midin-2-yl)-amino]-carbonyl]-amino]-sulfonyl]-methyl]-benzoesäuremethylester; Ethyl-2-{[(4-chlor-6-methoxy-2-pyrimidinyl)-aminocarbonyl]-aminosulfonyl}-benzoat; 2-Chlor-N-{[(4-methoxy-6-methyl-1,3,5-triazin-2-yl)-amino]-carbonyl}-benzolsulfonamid; 2-{[[((4-Methoxy-6-methyl-1,3,5-triazin-2-yl)-amino)-carbonyl]-amino]-sulfonyl}-benzoesäure oder deren Methylester; 3-[[[[(4-Methoxy-6-methyl-1,3,5-triazin-2-yl)-amino]-carbonyl]-amino]-sulfonyl]-thiophen-2-carbonsäure-methylester; N,S-Diethyl-N-cyclohexyl-thiolcarbamat; N,N-Di-n-propyl-thiocarbamidsäure-S-ethylester; S-Ethyl-N,N-hexamethylen-thiolcarbamat; S-[(4-Chlorphenyl)-methyl]-N,N-diethyl-thiocarbamat; N,N-Diisopropyl-S-(2,3,3-trichlorallyl)-thiolcarbamat; 4-Amino-6-t-butyl-3-ethylthio-1,2,4-triazin-5(4H)-on und 1-Methyl-3-phenyl-5-(3-trifluormethylphenyl)-4-pyridon. Einige dieser Mischungen zeigen auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

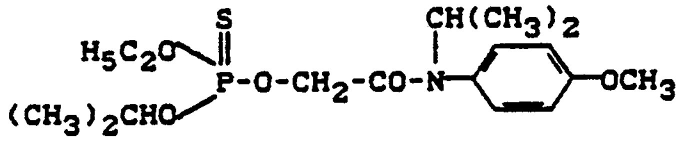

5,1 g (0,025 Mol) Thiophosphorsäure-chlorid-O-ethylester-O-isopropylester werden bei 10 °C bis 20 °C zu einer Mischung aus 5,6 g (0,025 Mol) N-Isopropyl-N-(4-methoxy-phenyl)-hydroxyessigsäureamid, 1,7 g (0,03 Mol) Kaliumhydroxid-Pulver und 50 ml Isopropanol unter Rühren tropfenweise gegeben. Das Reaktionsgemisch wird 15 Stunden bei 20 °C gerührt, dann mit 100 ml Methylenchlorid verdünnt, mit Wasser gewaschen, mit Magnesiumsulfat getrocknet und filtriert. Vom Filtrat wird das Lösungsmittel im Wasserstrahlvakuum abdestilliert, der Rückstand durch Verreiben mit Petrolether zur Kristallisation gebracht und das Produkt durch Absaugen isoliert.

Man erhält 7,5 g (80 % der Theorie) N-Isopropyl-N-(4-methoxy-phenyl)-O-(ethoxy-isopropoxy-thiophosphoryl)-oxyessigsäureamid vom Schmelzpunkt 63 °C.

Beispiel 2

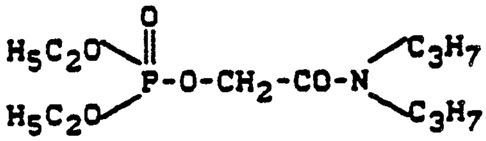

4,3 g (0,025 Mol) Phosphorsäure-chlorid-diethylester werden bei -10 °C bis 0 °C zu einer Mischung aus 4,0 g (0,025 Mol) N,N-Dipropyl-hydroxyessigsäureamid, 2,8 g (0,025 Mol) Kalium-tert-butylat und 75 ml Isopropanol unter Rühren tropfenweise gegeben. Das Reaktionsgemisch wird 15 Stunden bei 20 °C gerührt, dann mit 150 ml Methylenchlorid verdünnt, mit Wasser gewaschen, mit Magnesiumsulfat getrocknet und filtriert. Vom Filtrat wird das Lösungsmittel im Wasserstrahlvakuum sorgfältig abdestilliert.

Man erhält 7,1 g (96 % der Theorie) N,N-Dipropyl-O-diethoxyphosphoryl-oxyessigsäureamid als öligen Rückstand vom Brechungsindex $n_D^{20}$ = 1,4476.

Analog Beispiel 1 und 2 und entsprechend der allgemeinen Beschreibung des erfindungsgemäßen Herstellungsverfahrens können auch die in der nachstehenden Tabelle 3 aufgeführten Verbindungen der Formel (I) hergestellt werden.

**Tabelle 3: Beispiele für die Verbindungen der Formel (I)**

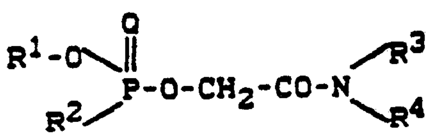

(I)

Tabelle 3

| Bsp. Nr. | Q | $R^1$ | $R^2$ | $R^3$ | $R^4$ | Schmelzpunkt bzw. Brechungsindex |
|---|---|---|---|---|---|---|
| 3 | S | $C_2H_5$ | $OC_2H_5$ | $CH_3$ | $CH_3$ | $n_D^{20}$ = 1,4791 |
| 4 | S | $CH_3$ | $OCH_3$ | $C_2H_5$ | $C_2H_5$ | $n_D^{20}$ = 1,4772 |
| 5 | S | $C_2H_5$ | $OC_2H_5$ | $C_2H_5$ | $C_2H_5$ | $n_D^{20}$ = 1,4678 |
| 6 | S | $CH_3$ | $OCH_3$ | $C_3H_7$ | $C_3H_7$ | $n_D^{20}$ = 1,4780 |
| 7 | S | $C_2H_5$ | $OC_2H_5$ | $C_3H_7$ | $C_3H_7$ | $n_D^{20}$ = 1,4755 |
| 8 | S | $C_2H_5$ | $OCH(CH_3)_2$ | $C_3H_7$ | $C_3H_7$ | $n_D^{20}$ = 1,4705 |
| 9 | S | $CH_3$ | $OCH_3$ | $CH(CH_3)_2$ | $CH(CH_3)_2$ | $n_D^{20}$ = 1,4795 |
| 10 | S | $C_2H_5$ | $OC_2H_5$ | $CH(CH_3)_2$ | $CH(CH_3)_2$ | $n_D^{20}$ = 1,4960 |
| 11 | S | $CH_3$ | $OCH_3$ | $C_4H_9$ | $C_4H_9$ | $n_D^{20}$ = 1,4779 |
| 12 | S | $C_2H_5$ | $SC_3H_7$ | $C_2H_5$ | $C_2H_5$ | $n_D^{20}$ = 1,5071 |
| 13 | S | $C_2H_5$ | $SC_3H_7$ | $C_3H_7$ | $C_3H_7$ | $n_D^{20}$ = 1,5045 |
| 14 | S | $C_2H_5$ | $SC_3H_7$ | $C_4H_9$ | $C_4H_9$ | $n_D^{20}$ = 1,4988 |
| 15 | S | $CH_3$ | $OCH_3$ | $CH_2CH{=}CH_2$ | $CH_2CH{=}CH_2$ | $n_D^{20}$ = 1,4940 |
| 16 | S | $C_2H_5$ | $OC_2H_5$ | $CH_2CH{=}CH_2$ | $CH_2CH{=}CH_2$ | $n_D^{20}$ = 1,4936 |

Tabelle 3 - Fortsetzung

| Bsp.-Nr. | Q | $R^1$ | $R^2$ | $R^3$ | $R^4$ | Schmelzpunkt bzw. Brechungsindex |
|---|---|---|---|---|---|---|
| 17 | S | $C_2H_5$ | $OCH(CH_3)_2$ | $CH_2CH{=}CH_2$ | $CH_2CH{=}CH_2$ | $n_D^{20} = 1,4886$ |
| 18 | S | $C_2H_5$ | $SC_3H_7$ | $CH_3$ | $C_4H_9$ | $n_D^{20} = 1,5100$ |
| 19 | S | $CH_3$ | $OCH_3$ | $CH_3$ | 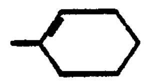 | $n_D^{20} = 1,5071$ |
| 20 | S | $C_2H_5$ | $OC_2H_5$ | $CH_3$ | 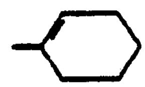 | $n_D^{20} = 1,4989$ |
| 21 | S | $CH_3$ | $OCH_3$ | $CH_3$ | $CH_2OCH_3$ | $n_D^{20} = 1,4845$ |
| 22 | S | $C_2H_5$ | $OC_2H_5$ | $CH(CH_3)CH_2CH_3$ | $OCH_3$ | $n_D^{20} = 1,4678$ |
| 23 | S | $CH_3$ | $OCH_3$ | $CH_3$ | 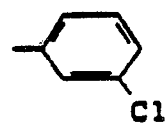 | 30 °C |
| 24 | S | $CH_3$ | $OCH_3$ | $CH_3$ | 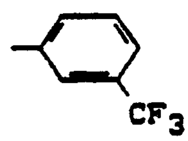 | $n_D^{20} = 1,4980$ |
| 25 | S | $C_2H_5$ | $OC_2H_5$ | $CH_3$ | 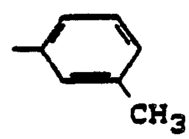 | $n_D^{20} = 1,5258$ |
| 26 | S | $C_2H_5$ | $OC_2H_5$ | $CH_3$ | 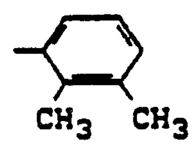 | 40 °C |

Tabelle 3 - Fortsetzung

| Bsp.-Nr. | Q | $R^1$ | $R^2$ | $R^3$ | $R^4$ | Schmelzpunkt bzw. Brechungsindex |
|---|---|---|---|---|---|---|
| 27 | S | $C_2H_5$ | $OC_2H_5$ | $CH_3$ | 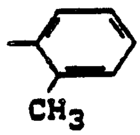 | $n_D^{20}$ = 1,5160 |
| 28 | S | $C_2H_5$ | $OC_2H_5$ | $CH_3$ | 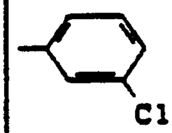 | $n_D^{20}$ = 1,5330 |
| 29 | S | $C_2H_5$ | $SC_3H_7$ | $CH_3$ | 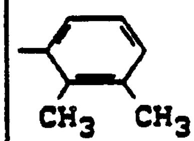 | $n_D^{20}$ = 1,5481 |
| 30 | S | $CH_3$ | $OCH_3$ | $CH(CH_3)_2$ | 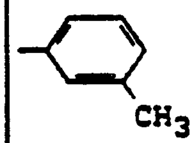 | 84 °C |
| 31 | S | $C_2H_5$ | $OC_2H_5$ | $CH(CH_3)_2$ | 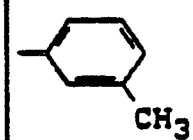 | 60 °C |
| 32 | S | $C_2H_5$ | $OCH(CH_3)_2$ | $CH(CH_3)_2$ | 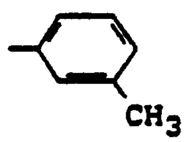 | 52 °C |
| 33 | S | $CH_3$ | $OCH_3$ | $CH(CH_3)_2$ | 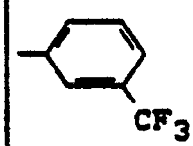 | 66 °C |
| 34 | S | $C_2H_5$ | $OC_2H_5$ | $CH(CH_3)_2$ | 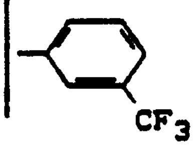 | 76 °C |

Tabelle 3 - Fortsetzung

| Bsp.-Nr. | Q | $R^1$ | $R^2$ | $R^3$ | $R^4$ | Schmelzpunkt bzw. Brechungsindex |
|---|---|---|---|---|---|---|
| 35 | S | $C_2H_5$ | $OCH(CH_3)_2$ | $CH(CH_3)_2$ | 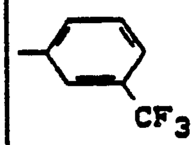 | 54 °C |
| 36 | S | $CH_3$ | $OCH_3$ | $CH(CH_3)_2$ | 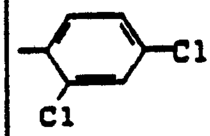 | (amorph) |
| 37 | S | $C_2H_5$ | $OC_2H_5$ | $CH(CH_3)_2$ | 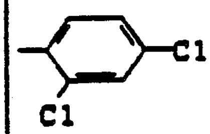 | 75 °C |
| 38 | S | $CH_3$ | $OCH_3$ | $CH(CH_3)_2$ | 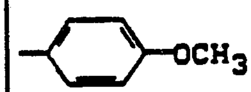 | 54 °C |
| 39 | S | $C_2H_5$ | $OC_2H_5$ | $CH(CH_3)_2$ | 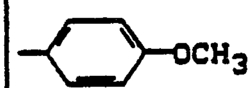 | 72 °C |
| 40 | S | $CH_3$ | $OCH_3$ | $CH(CH_3)_2$ | 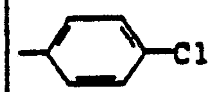 | 79 °C |
| 41 | S | $C_2H_5$ | $OC_2H_5$ | $CH(CH_3)_2$ | 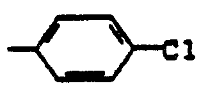 | 108 °C |
| 42 | S | $CH_3$ | $OC_3H_7$ | $CH(CH_3)_2$ | 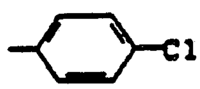 | 61 °C |
| 43 | O | $C_2H_5$ | $OC_2H_5$ | $CH(CH_3)_2$ | 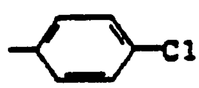 | 99 °C |
| 44 | O | $C_2H_5$ | $OC_2H_5$ | $CH_2CH=CH_2$ | $CH_2CH=CH_2$ | $n_D^{20}$ = 1,4652 |

**Tabelle 3** - Fortsetzung

| Bsp.-Nr. | Q | $R^1$ | $R^2$ | $R^3$ | $R^4$ | Schmelzpunkt bzw. Brechungsindex |
|---|---|---|---|---|---|---|
| 45 | S | $-CH_2CH_2CH_2O-$ | | $CH(CH_3)_2$ | 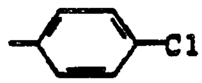 | 107 °C |
| 46 | S | $C_2H_5$ | $OCH(CH_3)_2$ | $CH(CH_3)_2$ | 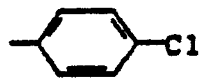 | 92 °C |
| 47 | S | $-CH_2CH_2CH_2O-$ | | $CH(CH_3)_2$ | 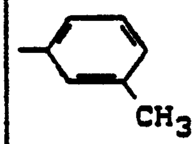 | 115 °C |
| 48 | S | $-CH_2CH_2CH_2O-$ | | $CH(CH_3)_2$ | 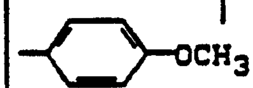 | 102 °C |
| 49 | S | $-CH_2CH_2CH_2O-$ | | $CH(CH_3)_2$ | 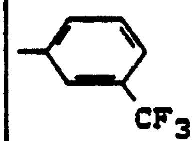 | 98 °C |
| 50 | S | $-CH_2CH_2CH_2O-$ | | $CH(CH_3)_2$ | 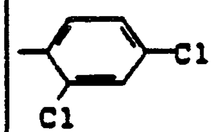 | 97 °C |
| 51 | S | $-CH_2CH_2CH_2O-$ | | $CH_2CH=CH_2$ | $CH_2CH=CH_2$ | (amorph) |
| 52 | S | $CH_3$ | $OCH_3$ | $CH_2OCH_3$ | 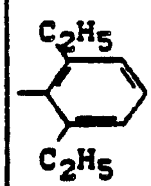 | (amorph) |
| 53 | S | $CH_3$ | $OCH_3$ | $CH_3$ | 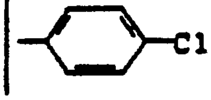 | $n_D^{20}$ = 1,5484 |

Tabelle 3 - Fortsetzung

| Bsp.-Nr. | Q | $R^1$ | $R^2$ | $R^3$ | $R^4$ | Schmelzpunkt bzw. Brechungsindex |
|---|---|---|---|---|---|---|
| 54 | S | $C_2H_5$ | $OCH(CH_3)_2$ | $CH(CH_3)_2$ | 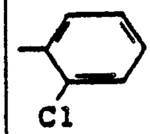 | |
| 55 | S | $CH_3$ | $OCH_3$ | $C_2H_5$ | 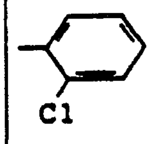 | |
| 56 | S | $C_2H_5$ | $OC_2H_5$ | $C_2H_5$ | 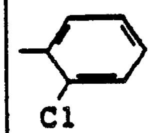 | |
| 57 | S | $CH_3$ | $OCH_3$ | $CH(CH_3)_2$ | 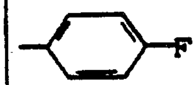 | |
| 58 | S | $CH_3$ | $OCH_3$ | $CH(CH_3)_2$ | 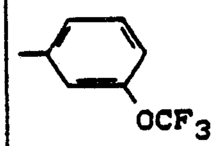 | 68° C |
| 59 | S | $CH_3$ | $OCH_3$ | $CH(CH_3)_2$ | 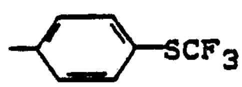 | |
| 60 | S | $CH_3$ | $OCH_3$ | $CH(CH_3)_2$ | 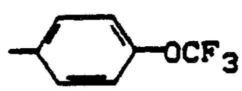 | |
| 61 | S | $CH_3$ | $OCH_3$ | $CH(CH_3)_2$ | 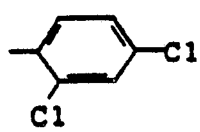 | |
| 62 | S | $CH_3$ | $OCH_3$ | $C_2H_5$ | 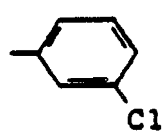 | |

Tabelle 3 - Fortsetzung

| Bsp.-Nr. | Q | $R^1$ | $R^2$ | $R^3$ | $R^4$ | Schmelzpunkt bzw. Brechungsindex |
|---|---|---|---|---|---|---|
| 63 | S | $CH_3$ | $OCH_3$ | $C_2H_5$ | 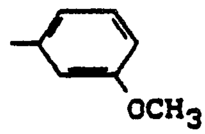 | |
| 64 | S | $C_2H_5$ | $OC_2H_5$ | $C_2H_5$ | 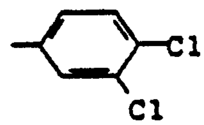 | |
| 65 | S | $CH_3$ | $OCH_3$ | $CH(CH_3)_2$ | 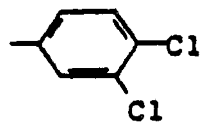 | 97° C |
| 66 | S | $CH_3$ | $OCH_3$ | $CH(CH_3)_2$ | 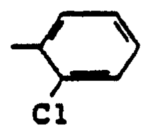 | |
| 67 | S | $CH_3$ | $OCH_3$ | $CH(CH_3)_2$ | 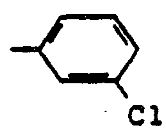 | |
| 68 | S | $C_2H_5$ | $OC_2H_5$ | $CH(CH_3)_2$ | 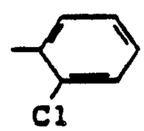 | |
| 69 | S | $C_2H_5$ | $OC_2H_5$ | $CH(CH_3)_2$ | 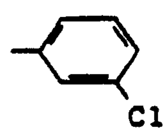 | |
| 70 | S | $C_2H_5$ | $OC_2H_5$ | $C_2H_5$ | 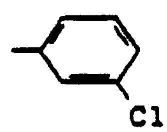 | |

Tabelle 3 - Fortsetzung

| Bsp.-Nr. | Q | $R^1$ | $R^2$ | $R^3$ | $R^4$ | Schmelzpunkt bzw. Brechungsindex |
|---|---|---|---|---|---|---|
| 71 | S | $CH_3$ | $OCH_3$ | $CH(CH_3)_2$ | 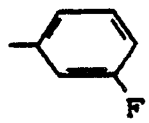 | 110° C |
| 72 | S | $C_2H_5$ | $OCH_3$ | $CH(CH_3)_2$ | 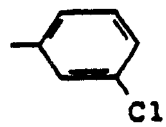 | 62° C |
| 73 | S | $C_2H_5$ | $OCH_3$ | $CH(CH_3)_2$ | 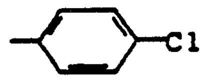 | 71° C |

Anwendungsbeispiel

Im folgenden Anwendungsbeispiel wird die Verbindung nachstehender Formel als Vergleichssubstanz verwendet:

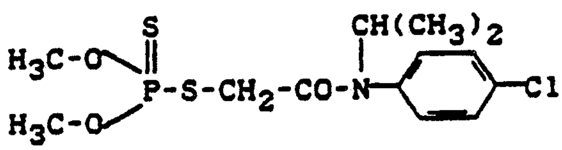 (A)

N-(4-Chlor-phenyl)-N-isopropyl-S-(dimethoxy-thiophosphoryl)-mercaptoessigsäureamid (Anilofos) (bekannt aus DE-OS 26 04 224).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (23), (40) und (41) deutlich stärkere Wirkung als die Vergleichssubstanz (A).

## Ansprüche

1. (Thio)Phosphoryloxyessigsäureamide der allgemeinen Formel (I)

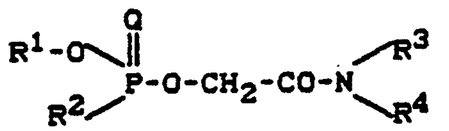

(I)

in welcher
Q für Sauerstoff oder Schwefel steht,
$R^1$ für gegebenenfalls durch Halogen substituiertes $C_1$-$C_6$-Alkyl oder Phenyl steht,
$R^2$ für gegebenenfalls durch Halogen substituiertes $C_1$-$C_6$-Alkyl oder Phenyl, für gegebenenfalls durch Halogen substituiertes $C_1$-$C_6$-Alkoxy oder Phenoxy oder für gegebenenfalls durch Halogen substituiertes $C_1$-$C_6$-Alkylthio oder Phenylthio steht, oder die beiden Gruppierungen
O-$R^1$ und $R^2$ zusammen für gegebenenfalls verzweigtes $C_2$-$C_5$-Alkylendioxy stehen,
$R^3$ für gegebenenfalls durch Halogen oder $C_1$-$C_4$-Alkoxy substituiertes $C_1$-$C_6$-Alkyl, für gegebenenfalls durch Halogen substituiertes $C_2$-$C_6$-Alkenyl oder für $C_2$-$C_6$-Alkinyl steht und
$R^4$ für gegebenenfalls durch Halogen oder $C_1$-$C_4$-Alkoxy substituiertes $C_1$-$C_6$-Alkyl, für gegebenenfalls durch Halogen substituiertes $C_2$-$C_6$-Alkenyl, für $C_2$-$C_6$-Alkinyl, für $C_5$- oder $C_6$-Cycloalkenyl, für gegebenenfalls durch $C_1$-$C_4$-Alkoxy substituiertes $C_1$-$C_6$-Alkoxy, für $C_3$-$C_4$-Alkenyloxy oder für durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio und/oder $C_1$-$C_2$-Halogenalkylthio substituiertes Phenyl steht.

2. (Thio)Phosphoryloxyessigsäureamide der Formel (I) gemäß Anspruch 1, dadurch gekennzeichnet, daß darin
Q für Sauerstoff oder Schwefel steht,
$R^1$ für gegebenenfalls durch Fluor und/oder Chlor substituiertes $C_1$-$C_4$-Alkyl oder Phenyl steht,
$R^2$ für gegebenenfalls durch Fluor und/oder Chlor substituiertes $C_1$-$C_4$-Alkyl oder Phenyl, für gegebenenfalls durch Fluor und/oder Chlor substituiertes $C_1$-$C_4$-Alkoxy oder Phenoxy oder für gegebenenfalls durch Fluor und/oder Chlor substituiertes $C_1$-$C_4$-Alkylthio oder Phenylthio steht, oder die beiden Gruppierungen
O-$R^1$ und $R^2$ zusammen für Ethan-1,2-dioxy (Ethylendioxy) oder Propan-1,3-dioxy (Propylendioxy) stehen,
$R^3$ für gegebenenfalls durch Fluor, Chlor oder $C_1$-$C_3$-Alkoxy substituiertes $C_1$-$C_4$-Alkyl, für gegebenenfalls durch Fluor oder Chlor substituiertes $C_3$-$C_4$-Alkenyl oder für $C_3$-$C_4$-Alkinyl steht und
$R^4$ für gegebenenfalls durch Fluor, Chlor oder $C_1$-$C_3$-Alkoxy substituiertes $C_1$-$C_4$-Alkyl, für gegebenenfalls durch Fluor oder Chlor substituiertes $C_3$-$C_4$-Alkenyl, für $C_3$-$C_4$-Alkinyl, für $C_5$- oder $C_6$-Cycloalkenyl, für gegebenenfalls durch $C_1$-$C_3$-Alkoxy substituiertes $C_1$-$C_4$-Alkoxy, für $C_3$-$C_4$-Alkenyloxy oder für durch Fluor, Chlor, Brom, $C_1$-$C_3$-Alkyl, Trifluormethyl, $C_1$-$C_3$-Alkoxy, $C_1$-$C_2$-Fluoralkoxy, $C_1$-$C_2$-Chlorfluoralkoxy, $C_1$-$C_3$-Alkylthio, $C_1$-$C_2$-Fluoralkylthio und/oder $C_1$-$C_2$-Chlorfluoralkylthio substituiertes Phenyl steht.

3. (Thio)Phosphoryloxyessigsäureamide der Formel (I) gemäß Anspruch 1, dadurch gekennzeichnet, daß darin
Q für Sauerstoff oder Schwefel steht,
$R^1$ für Methyl, Ethyl, Propyl oder Isopropyl steht,
$R^2$ für Methoxy, Ethoxy, Propoxy, Isopropoxy, Methylthio, Ethylthio, Propylthio oder Isopropylthio steht, oder die beiden Gruppierungen
O-$R^1$ und $R^2$ zusammen für Propan-1,3-dioxy (Propylendioxy) stehen,
$R^3$ für Methyl, Ethyl, Propyl, Isopropyl, Butyl, Isobutyl, Methoxymethyl, Methoxyethyl, Methoxypropyl, Ethoxymethyl, Propoxymethyl, Isopropoxymethyl, Ethoxyethyl, Propoxyethyl, Isopropoxyethyl, Allyl oder Propargyl steht und
$R^4$ für Methyl, Ethyl, Propyl, Isopropyl, Butyl, Isobutyl, Methoxyethyl, Ethoxyethyl, Propoxyethyl, Isopropoxyethyl, Allyl, Propargyl, Cyclohexenyl, Methoxy, Ethoxy, Propoxy, Isopropoxy, Butoxy, Isobutoxy, Methoxyethoxy, Ethoxyethoxy oder für durch Fluor, Chlor, Brom, Methyl, Ethyl, Trifluormethyl, Methoxy, Ethoxy, Difluormethoxy, Trifluormethoxy, Chlordifluormethoxy, Methylthio, Ethylthio, Trifluormethylthio und/oder Chlordifluormethylthio substituiertes Phenyl steht.

4. (Thio)Phosphoryloxyessigsäureamide der Formel (I) gemäß Anspruch 1, dadurch gekennzeichnet, daß darin

(a) Q für Schwefel, $R^1$, $R^2$ und $R^3$ jeweils für Methyl stehen und $R^4$ für 3-Chlorphenyl steht, oder

(b) Q für Schwefel steht, $R^1$ und $R^2$ für Methyl stehen, $R^3$ für Isopropyl steht und $R^4$ für 4-Chlorphenyl steht, oder

(c) Q für Schwefel steht, $R^1$ und $R^2$ für Ethyl stehen, $R^3$ für Isopropyl steht und $R^4$ für 4-Chlorphenyl steht.

5. Verfahren zur Herstellung von (Thio)Phosphoryloxyessigsäureamiden der Formel (I) gemäß Anspruch 1, dadurch gekennzeichnet, daß man (Thio)Phosphorylhalogenide der allgemeinen Formel (II)

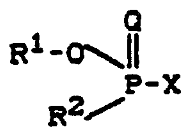

(II)

in welcher
Q, $R^1$ und $R^2$ die in Anspruch 1 angegebenen Bedeutungen haben und
X für Halogen steht,
mit Hydroxyessigsäureamiden der allgemeinen Formel (III)

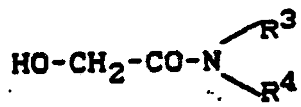

(III)

in welcher
$R^3$ und $R^4$ die in Anspruch 1 angegebenen Bedeutungen haben,
gegebenenfalls in Gegenwart eines Verdünnungsmittels und gegebenenfalls in Gegenwart eines Säurebindemittels umsetzt.

6. Herbizide Mittel, gekennzeichnet durch einen Gehalt an (Thio)Phosphoryloxyessigsäureamiden der Formel (I) gemäß Anspruch 1.

7. Verfahren zur Bekämpfung von unerwünschtem Pflanzenwachstum, dadurch gekennzeichnet, daß man (Thio)Phosphoryloxyessigsäureamide der Formel (I) gemäß Anspruch 1 auf die unerwünschten Pflanzen oder ihren Lebensraum einwirken läßt.

8. Verwendung von (Thio)Phosphoryloxyessigsäureamiden der Formel (I) gemäß Anspruch 1 zur Bekämpfung von Pflanzenwachstum.

9. Verfahren zur Herstellung von herbiziden Mitteln, dadurch gekennzeichnet, daß man (Thio)-Phosphoryloxyessigsäureamide der Formel (I) gemäß Anspruch 1 mit Streckmitteln und/oder oberflächenaktiven Mitteln vermischt.